# EUROPÄISCHE PATENTANMELDUNG

(11) **EP 4 477 144 A2**
(43) Veröffentlichungstag der Anmeldung: **18.12.2024**
(21) Anmeldenummer: 24209429.0
(22) Anmeldetag: 20.08.2019
(51) Int. Cl.: A61B 5/08

(54) **TELEMONITORING IN DER BEATMUNG**

(30) Priorität: 14.09.2018 DE 102018007256
(62) Teilanmeldung aus: 19000379.8
(71) Anmelder: Löwenstein Medical Technology S.A., 2557 Luxembourg (LU)
(72) Erfinder: Schwaibold, Matthias, 76228 Karlsruhe (DE)
(74) Vertreter: Löwenstein Medical IP

(57) **Zusammenfassung**

Die vorliegende Erfindung betrifft System (10) für die Beatmung umfassend zumindest ein Beatmungsgerät (20), zumindest eine Eingabeeinheit (30) und zumindest eine Telemonitoringeinheit (60), wobei das mindestens eine Beatmungsgerät (20) mindestens eine Sensoreinheit (21), eine Aufbereitungseinheit (22), eine Berechnungseinheit (23), eine Erkennungseinheit (24), eine Speichereinheit (25) und eine Kommunikationseinheit (27) umfasst, wobei die Sensoreinheit (21) Sensoren umfasst, deren Messwerte eine Ermittlung von Druck und/oder Flow und/oder Volumen der zugeführten und/oder ausgeatmeten Gase erlauben, die Aufbereitungseinheit (22) die erfassten Messwerte aufbereitet, die Berechnungseinheit (23) basierend auf den Messwerten Signale und/oder Kenngrößen bestimmt und die Speichereinheit (25) diese Werte speichert und die Erkennungseinheit (24), die Werte zu weiteren Analysen nutzt, wobei die Eingabeeinheit (30) eingerichtet ist, durch einen Anwender (A) des Systems (10) über die Eingabeeinheit (30) eingegebene Werte und Informationen des Anwenders (A) zu seinem Gesundheitszustand dem System (10) bereitzustellen, und wobei die Kommunikationseinheit (26) die Werte selbsttätig, ggf. wiederholt oder dauerhaft, nach einer oder mehreren fest einprogrammierten und/oder frei eingegebenen Zeitintervallen an die Telemonitoringeinheit (60) übermittelt, wobei die Telemonitoringeinheit (60) eingerichtet ist, die übermittelten Werte und/oder Informationen des Beatmungsgerätes (20) und der Eingabeeinheit (30) zu empfangen, zu speichern, zu analysieren und/oder zu bewerten.

## Beschreibung

Die Erfindung betrifft ein System für die Beatmung. Herkömmliche Vorrichtungen für die Beatmung weisen zumeist ein Display auf, um bestimmte aktuelle Informationen für den Anwender darzustellen. Im einfachsten Fall werden Parameter der Beatmung, wie aktueller Fluss oder Druck der Atemgase in Form von Zahlenwerten oder in Form graphischer Darstellungen angezeigt. Daneben sind andere optische Anzeigemöglichkeiten wie Balkendiagramme zur Darstellung von Parametern bekannt.

Im Stand der Technik sind zudem Vorrichtung für die Beatmung bekannt, bei denen Parameter innerhalb eines Beatmungsgerätes analysiert werden und auf dem Beatmungsgerät dem Anwender dargestellt werden. Auch ist es bekannt, Parameter und/oder Messwerte beispielsweise in einem internen Speicher in dem Beatmungsgerät zu speichern oder auf einem an das Beatmungsgerät angeschlossenen USB Stick zu speichern, sodass die Messwerte auf dem Speichergerät an einen Betreuer weitergegeben werden können. Optional können Messwerte zur Speicherung auch an einen Cloudspeicher übermittelt werden.

Die im Stand der Technik bekannten Vorrichtungen für die Beatmung weisen jedoch den Nachteil auf, dass nur der Anwender und nur auf dem Beatmungsgerät eine Analyse der erfassten Messwerte angezeigt bekommt. Da ein Anwender jedoch keine Beurteilung der angezeigten und/oder analysierten Messwerte vornehmen kann, fallen somit schlechte Messergebnisse erst durch eine Sichtung der auf dem USB Stick oder in der Cloud abgespeicherten Werte durch den Betreuer, wenn dieser vor Ort ist, auf. Dies hat zur Folge, dass ein Anwender ggf. über einen langen Zeitraum schlecht therapiert wird.

Neben der Überwachung der Messwerte ist auch die Überwachung der Funktionen und Einstellungen des Beatmungsgerätes erschwert. Da der Anwender basierend auf den Analysen keine Rückschlüsse auf schlechte Einstellungen am Beatmungsgerät ziehen kann, ist er auf eine Beurteilung der Analysen sowie auf eine darauf basierende Einstellung des Beatmungsgerätes durch einen Betreuer angewiesen. Da der Betreuer jedoch nur in zeitlichen Abständen vor Ort ist, kann dieser nur dann Änderungen an den Einstellungen des Beatmungsgerätes vornehmen, um auf die Qualität der Therapie des Anwenders einwirken zu können.

Des Weiteren beziehen sich die Analysen der bisher bekannten Vorrichtungen lediglich auf durch ein Beatmungsgerät erfasste Messwerte oder auf durch den Anwender über ein Display eingegebene Daten, wie beispielsweise Alter und Gewicht. Eine Berücksichtigung weiterer personenbezogener Daten, Messwerte und Informationen, insbesondere ärztliche Fachinformationen und durch einen Arzt erfasste Messwerte oder Vergleichswerte, bleiben bei den bisherigen Analysen bzw. Auswertungen unbeachtet.

US 2018/0184945 A1 beschreibt ein Verfahren zum Erkennen einer Verschlechterung des kardiopulmonalen Zustands eines mithilfe eines Beatmungsgeräts behandelten Patienten. Das Verfahren kann auf dem Beatmungsgerat und/oder auf einem Server, der bestimmte Beatmungsparameter vom Beatmungsgerat empfangen kann, implementiert sein.

US 2018/0082033 A1 beschreibt ein Verfahren zum Steuern eines Beatmungsgeräts, bei dem eine Änderung eines Aktivitätsniveaus eines Benutzers auf der Grundlage von Daten, die von einem in der Nähe des Benutzers befindlichen Sensor gesammelt werden, erkannt wird, bei dem die Notwendigkeit einer Änderung einer Einstellung eines Parameters des mit dem Benutzer verbundenen Beatmungsgeräts als Reaktion auf die erkannte Änderung des Aktivitätsniveaus erkannt wird und bei dem die Einstellung des Parameters entsprechend dem Aktivitätsniveau geändert wird.

WO 2018/083711 A1 beschreibt ein Beatmungssystem, das ein Beatmungsgerat, ein mit dem Beatmungsgerat verbundenes Mobilgerat zum Speichern und Anzeigen von Daten aus dem Beatmungsgerat und eine Verarbeitungs- und Speichereinheit zum Verarbeiten und Speichern von Daten aus dem Mobilgerat umfasst.

US 2016/0364617 A1 beschreibt ein Kamerasystem zum Überwachen einer schlafenden Person. Das Kamerasystem ermöglicht eine nicht invasive Bestimmung eines Aktivitätszustands der schlafenden Person mithilfe einer KI-gestützten Bildverarbeitung.

US 2013/0333702 A1 beschreibt ein Beatmungssystem mit einer Eingabe- und einer Anzeigeschnittstelle, auf der eine Rückmeldung beglich des Sitzes eines Patienteninterface angezeigt werden kann.

US 2017/0311879 A1 beschreibt ein Beatmungssystem mit einem Beatmungsgerat und einem Datenserver zur Datenkommunikation mit dem Beatmungsgerat. Der Datenserver kann einen Therapiequalitätsindikator aus Nutzerdaten bestimmen und an eine dem jeweiligen Patienten zugeordnete Berechnungseinheit senden.

US 2012/0240933 A1 beschreibt ein Beatmungssystem, das ein schlechtsitzendes Patienteninterface erkennen und entsprechende Korrekturen über eine Benutzerschnittstelle ausgeben kann.

Aufgabe der vorliegenden Erfindung ist es daher, ein System zur Beatmung anzugeben, dass die Überwachung und Qualität einer Beatmung verbessert.

Diese Aufgabe wird durch den Gegenstand mit den Merkmalen des Anspruchs 1 gelöst.

Gegenstand der vorliegenden Erfindung ist ein System für die Beatmung umfassend zumindest ein Beatmungsgerät, zumindest eine Eingabeeinheit und zumindest eine Telemonitoringeinheit, wobei das mindestens eine Beatmungsgerät mindestens eine Sensoreinheit, eine Aufbereitungseinheit, eine Berechnungseinheit, eine Erkennungseinheit, eine Speichereinheit und eine Kommunikationseinheit umfasst, wobei die Sensoreinheit Sensoren umfasst, deren Messwerte eine Ermittlung von Druck und/oder Flow und/oder Volumen der zugeführten und/oder ausgeatmeten Gase erlauben, die Aufbereitungseinheit die erfassten Messwerte aufbereitet, die Berechnungseinheit basierend auf den Messwerten Signale und/oder Kenngrößen bestimmt und die Speichereinheit diese Werte speichert und die Erkennungseinheit die Werte zu weiteren Analysen nutzt, wobei die Eingabeeinheit eingerichtet ist, durch einen Anwender des Systems über die Eingabeeinheit eingegebene Werte und Informationen des Anwenders zu seinem Gesundheitszustand dem System bereitzustellen, und wobei die Kommunikationseinheit die Werte an die Telemonitoringeinheit übermittelt. Als Werte werden zusammenfassend Messwerte, Signale, Kenngrößen und Informationen bezeichnet.

Die Aufbereitungseinheit des Beatmungsgerätes ist eingerichtet, die durch die Sensorik der Sensoreinheit erfassten Messwerte aufzubereiten. Eine Aufbereitung kann dabei eine Glättung, eine statistische Auswertung, eine Bestimmung eines Minimums oder eines Maximums, eines Mean und/oder Medians, eines Perzentils sowie die Erkennung von Mustern oder eine Über-/Unterschreitung von Grenzwerten sein. Weitere Signalaufbereitungen sind beispielsweise Heruntertaktung oder Artefaktbereinigung.

Die Berechnungseinheit des Beatmungsgerätes ist eingerichtet, aus den aufbereiteten Messwerten Signalen und/oder Kenngrößen zu ermitteln. Eine Kenngröße kann beispielsweise einen Mittelwert, ein Median, ein Perzentil, eine Ableitung, eine Häufigkeitsverteilung, eine Dauer oder ein Anteil des Über-oder Unterschreibens eines Schwellenwertes sein. Die Berechnungseinheit ist eingerichtet, die aufbereiteten und berechneten Kenngrößen an die Erkennungseinheit zu übersenden, wobei die Erkennungseinheit eingerichtet ist, Ereignisse und/oder Zustände zu erkennen. Derartige Ereignisse können Atemaussetzer, Artefakte, Hustenstöße, Sauerstoff-Entsättigungen, asynchrone zwischen Gerät und Patient, Einatmen, Ausatmen, mandatorische Atemzüge oder Alarme sein.

Die Speichereinheit des Beatmungsgerätes ist eingerichtet, die durch die Sensorik der Sensoreinheit erfassten Messwerte, die aufbereiteten Messwerte, Signale, Kenngrößen sowie die Ereignisse und Zustände, die durch die Erkennungseinheit erfasst wurden, zwischenzuspeichern.

Die Kommunikationseinheit des Beatmungsgerätes ist eingerichtet, die in der Speichereinheit zwischengespeicherten Messwerte, Signale und Kenngrößen an die Telemonitoringeinheit zu übermitteln.

Optional kann das Beatmungsgerät eine Überwachungseinheit umfassen, die eingerichtet ist, technische Probleme zu erkennen. Technische Probleme können dabei unter anderem sein: ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen des erlaubten Temperaturbereiches. Die Überwachungseinheit des Beatmungsgerätes kein eingerichtet sein, bei einer Erkennung eines technischen Problems, einen Alarm auszugeben, der dem Anwender/Patienten auf dem Beatmungsgerät angezeigt wird.

In der Regel umfasst das Beatmungsgerät zudem zumindest eine Druckerzeugung und ein Patienteninterface.

Erfindungsgemäß ist die Telemonitoringeinheit eingerichtet, die übermittelten Werte und/oder Informationen des Beatmungsgerätes und der Eingabeeinheit des Beatmungsgerätes zu empfangen, zu speichern, zu analysieren und/oder zu bewerten. Optional kann die Übertragung der Messwerte und/oder Informationen zeitgesteuert, manuell ausgelöst (z.B. am Heimtherapiegeräte oder am Server ausgelöst), ereignisgesteuert (z.B. bei Erkennung bestimmter kritischer Zustände durch das Therapiegerät) oder als dauerhafte Übertragung, zumindest während einer laufenden Therapie, eingerichtet sein. Vorzugsweise erfolgt eine Übertragung der Messwerte, Signale, Kenngrößen und/oder Informationen des Anwenders alle 2 Stunden bis 7 Tagen, insbesondere alle 1 bis 3 Tage. In einer bevorzugten Ausführung erfolgt die Übertragung zumindest einmal pro Tag/pro 24 Stunden. Alternativ kann die Kommunikationseinheit des Beatmungsgerätes eingerichtet sein, die Werte stundenweise zusammenzugefasst zu übertragen oder die Messwerte in Echtzeit zu übertragen. Optional ist der Übertragungszyklus durch den Anwender und/oder durch den Betreuer frei wählbar. Die Kommunikationseinheit des Beatmungsgerätes kann eingerichtet sein, die Übertragung selbsttätig, ggf. wiederholt oder dauerhaft, nach einer oder mehreren fest einprogrammierten und/oder frei eingegebenen Zeitintervallen durchzuführen. Optional wird zusätzlich analysiert, ob eine Datenübertragung erfolgreich stattgefunden hat, ob sie regelmäßig stattgefunden hat und/oder ob sie fehlerfrei erfolgte.

Im Falle eines Ausfalls einer Datenverbindung ist die Speichereinheit des Beatmungsgerätes eingerichtet, die Messwerte und/oder die Informationen aus der Eingabeeinheit zumindest einen Tag zu speichern, wobei die Kommunikationseinheit des Beatmungsgerätes eingerichtet ist, die Messwerte an die Telemonitoringeinheit zu übertragen, sobald eine Datenverbindung erneut aufgebaut wurde.

In der Regel werden die Informationen des Anwenders und die durch die Sensorik des Beatmungsgerätes erfassten Messwerte in der Telemonitoringeinheit zusammengeführt. Optional kann die Kommunikationseinheit des Beatmungsgerätes eingerichtet sein, die Informationen und Werte des Anwenders und die Messwerte, Kenngrößen und Signale des Beatmungsgerätes bereits im Beatmungsgerät zusammenzuführen und dann zusammen an die Telemonitoringeinheit zu übermitteln.

Die Telemonitoringeinheit bietet somit den Vorteil, dass die Werte und Informationen des Beatmungsgerätes und der Eingabeeinheit in der Telemonitoringeinheit kombinierbar und zusammen auswertbar sind. Die Eingabeeinheit kann als Bestandteil eines Mensch-Maschine-Interfaces des Beatmungsgerätes, einer Software für ein Smartphone, ein Tablett oder einen PC, einer Software für einen Sprachassistenten oder als eigenständige Elektronik mit einem Mensch-Maschine-Interfaces ausgebildet sein. Über die Eingabeeinheit kann somit ein Anwender Werte und Informationen zu seinem Gesundheitszustand in das erfindungsgemäße System für die Beatmung einbringen. Die Eingabeeinheit kann somit extern des Beatmungsgerätes ausgebildet sein. Alternativ ist die Eingabeeinheit in oder an dem Beatmungsgerät ausgebildet. Dies bietet den Vorteil, dass die Werte und Informationen zu seiner Person direkt durch den Anwender in das System einbringbar sind. Dadurch kann die Auswertung der erfassten Messwerte, Signale und oder Kenngrößen basierend auf zusätzlichen Messwerten des Anwenders bzw. basierend auf Informationen zum allgemeinen Gesundheitszustand des Anwenders erfolgen.

Typischerweise ist die Eingabeeinheit eingerichtet, die durch den Anwender eingegebenen Werte und/oder Informationen zu seinem Gesundheitszustand wahlweise an das Beatmungsgerät und/oder an die Telemonitoringeinheit zu übermitteln.

Die Eingabeeinheit kann getrennt von dem Beatmungsgerät ausgebildet sein. Durch die getrennte Anordnung ist eine räumliche Nähe des Anwenders zum Beatmungsgerät zur Eingabe von Werten und/oder Nationen nicht erforderlich.

Die in dem Beatmungsgerät ermittelten Messwerte und die über die Eingabeeinheit in das System eingegebenen Werte und/oder Informationen können getrennt voneinander an die Telemonitoringeinheit übermittelt werden. Alternativ kann die Eingabeeinheit eingerichtet sein, die Messwerte und/Informationen zunächst an das Beatmungsgerät zu ermitteln und , wobei die Werte und/oder Informationen dann über das Beatmungsgerät an die Telemonitoringeinheit übermittelt werden.

Die Telemonitoringeinheit ist typischerweise externen des Beatmungsgerätes angeordnet. Durch die externe Speicherung, Analyse und Bewertung der Werte und/oder Informationen kann ortsungebunden auf die Werte, Analysen und Bewertungen der Telemonitoringeinheit zugegriffen werden. Durch die externe Anordnung ist die Telemonitoringeinheit eingerichtet, sowohl Informationen von dem Beatmungsgerät als auch von einer wahlweise extern angeordneten Eingabeeinheit zu empfangen.

Die Telemonitoringeinheit kann ferner eingerichtet sein, die analysierten und/oder bewerteten Werte an einen Betreuer weiterzuleiten. Dies bietet den Vorteil, dass mittels der Telemonitoringeinheit ermöglicht wird, die durch das Beatmungsgerät erfassten Messwerte, die aufbereitet, berechnet und analysiert wurden und gegebenenfalls mit Werten und/oder Informationen, die durch den Anwender über die Eingabeeinheit in das System eingebracht wurden, verglichen wurden an einen Betreuer, vorzugsweise einen behandelnden Arzt, digital zu übermitteln. Dies ermöglicht eine Überwachung von Messwerten und der Funktion des Beatmungsgerätes, des Zustandes des Anwenders durch einen Arzt, der nicht vor Ort anwesend ist. Beispielsweise kann so der Arzt bei einer festgestellten Verschlechterung der Messwerte oder gegebenenfalls aufgrund ausgegebenen Alarme aktiv eine Rückmeldung an den Anwender geben beispielsweise kann der Arzt den Anwender über einen direkten Kommunikationskanal, beispielsweise via Telefon kontaktieren.

In Ausgestaltung umfasst die Telemonitoringeinheit eine Kommunikationseinheit, eine Speichereinheit, zumindest eine Analyseeinheit, zumindest eine Ausgabeeinheit, eine Bewertungseinheit und zumindest eine Informationseinheit.

Die Kommunikationseinheit der Telemonitoringeinheit ist als Puffer zur Kommunikation mit einer Vielzahl von Beatmungsgeräten eingerichtet. Die Kommunikationseinichtung ist eingerichtet, Messwerte und Information von mindestens einem Beatmungsgerät und/oder mindestens einer Eingabeeinheit zu empfangen. Optional kann die Kommunikationseinrichtung der Telemonitoringeinheit eingerichtet sein, Messwerte und/oder Informationen einer zusätzlichen Messeinheit oder einer zusätzlichen Telemonitoringeinheit zu empfangen.

Die Speichereinheit der Telemonitoringeinheit ist eingerichtet, die mittels der Kommunikationseinheit empfangenen Werte und Werte, die über die Informationseinheit dem System zur Verfügung gestellt werden, zu speichern und gegebenenfalls eine Zuordnung eines Gerätes seine Patienten vorzunehmen, beispielsweise über einen Namen des Patienten, eine ID oder eine Seriennummer. Die Speichereinheit ist somit eingerichtet, die von der zusätzlichen Messeinheit und der zusätzlichen Telemonitroingeinheit, der Eingabeeinheit und von dem Beatmungsgerät ermittelte Messwerte und Informationen zumindest zwischenzuspeichern. Die Speichereinheit ist eingerichtet, die gespeicherten Werte zumindest einer Analyseeinheit bereitzustellen.

Vorzugsweise ist jeweils eine Analyseeinheit für zumindest eine Fragestellung eingerichtet. Beispielsweise können die folgenden Fragestellungen beantwortet werden: Wir das Beatmungsgerät ausreichend genutzt? Ist die Dichtigkeit von Mund und Patienteninterface ausreichend? Funktioniert das Beatmungsgerät richtig? Sind die Konfigurationen/Einstellungen am Beatmungsgerät richtig gewählt? Ist der Krankheitszustand stabil ohne Anzeichen einer Exazerbation/Dekompensation? Die Fragestellungen sind frei wählbar und können durch weitere Fragestellungen ergänzt werden. Typischerweise ist eine Auswahl an Fragestellung in der Telemonitoringeinheit hinterlegt, wobei die durch Analyseeinheiten auszuwertenden Fragestellungen je nach Anwender durch einen Betreuer frei zusammenstellbar sind. Die Analyse erfolgt vorzugsweise anhand von Regeln, Schwellenwerten oder künstlicher Intelligenz. Dabei erfolgt eine Gewichtung der empfangenen Informationen je nach Fragestellung. Die Analyseeinheiten können eingerichtet sein, Werte je nach Quellort, beispielsweise von dem Betreuer oder dem Anwender stammend, zu gewichten. Alternativ können die Analyseeinheiten eingerichtet sein, die Fragestellungen gemäß hinterlegbarer Regeln zu gewichten.

In einer bevorzugten Ausführungsform umfasst das erfindungsgemäße System mindestens vier Analyseeinheiten, wobei jede der Analyseeinheiten eine Fragestellung repräsentiert. Die voreingestellten Logiken der Fragestellungen in den jeweiligen Analyseeinheiten sind veränderbar.

In der Regel ist jeweils eine Ausgabeeinheit eingerichtet, für eine durch die Analyseeinheit analysierte Fragestellung ein Ergebnis auszugeben. Das Ergebnis kann vorzugsweise in Form einer Kennzahl oder einer Grafik und/oder durch farbliche Differenzierung dargestellt werden. Vorzugsweise wird das Ergebnis als erfüllt oder nicht erfüllt bzw. gut oder schlecht ausgegeben. Typischerweise umfasst die Telemonitoringeinheit zumindest zwei Analyseeinheiten. Über die Ausgabeeinheiten kann eine Erkennungsmeldung von suboptimalen Einstellungen des Beatmungsgerätes oder Beatmungssituationen, die die Therapiequalität negativ beeinflussen können, erfolgen. Beispielsweise kann über die Ausgabeeinheit eine Information über eine schlechte Trigger Einstellung/Asynchronie, eine Information über Air Traps, beispielsweise über einen intrinsischen PEEP bei einer Auswertung der Flusskurve, eine Information über eine starke Sekretbildung, beispielsweise weinrauschender Flusskurse oder die Häufigkeit von Hustenstößen, eine Information über eine Unregelmäßigkeit oder nicht sinnvolle Nutzungspatterns von Beatmungsprogrammen oder Sonderfunktion, eine Information über die Alterung des Akkus oder eine Information über falsch angeschlossenes Zubehör ausgegeben werden. Vorzugsweise umfasst die Telemonitoringeinheit jeweils eine Anzahl an Ausgabeeinheiten, die der Anzahl an Analyseeinheiten entspricht. In einer vorteilhaften Ausführungsform umfasst die Telemonitoringeinheit vier Analyseeinheiten und vier entsprechende Ausgabeeinheiten.

Die Bewertungseinheit ist in der Regel eingerichtet, eine Gesamtbewertung für einen Patienten basierend auf den durch die Ausgabeeinheiten ermittelten Ergebnisse für einen Anwender auszugeben. Die Gesamtbewertung ist eingerichtet, anhand der durch die Ausgabeeinheiten ausgegebenen Ergebnisse eine Gesamtbewertung des Gesundheitszustandes des Anwenders auszugeben. Die Gesamtbewertung kann anhand der Anzahl eventueller Probleme dargestellt werden. Bei einer kritischen Gesamtbewertung kann eine aktive Benachrichtigung des Betreuers per Chat, E-Mail, SMS oder Anruf erfolgen.

Die Bewertungseinheit ist eingerichtet, basierend auf zumindest einer erfüllten Fragestellung eine Prädiktion über den Verlauf der Messwerte, den Gesundheitszustand oder mögliche auftretende Komplikationen auszugeben. In einer vorteilhaften Ausführung wird basierend auf zumindest zwei als erfüllt bewerteten Fragestellungen eine Prädiktion über den Gesundheitszustand oder auftretende Komplikationen ausgegeben. Beispielsweise kann eine Prädiktion zum Gesundheitszustand ausgegeben werden, wenn zumindest zwei von vier Fragestellungen als erfüllt ausgegeben werden. Optional kann eine Prädiktion auch für weitere Kategorien erfolgen. Beispielsweise kann eine Prädiktion in Bezug auf einen Parameter wie beispielsweisen einen Temperaturverlauf ausgegeben werden. Eine Prädiktion kann beispielsweise auch eine Information über eine zunehmend schnellere und flachere Atmung sein, die beispielsweise aus Trends des Tidalvolumens, der Atemfrequenz, des alveolärem Tidalvolumens oder dem Rapid Shallow Breathing Index ermittelt wird. Alternativ ist die Bewertungseinheit eingerichtet, basierend auf zumindest einer nicht erfüllten Fragestellung eine Prädiktion über den Verlauf der Messwerte, den Gesundheitszustand oder mögliche auftretende Komplikationen auszugeben. In der Regel ist die Ausgabe der Prädiktion basierend auf einer Erfüllung oder Nicht-Erfüllung wählbar.

Die Bewertungseinheit ist eingerichtet, basierend auf der Prädiktion, beispielsweise einer Komplikation, einen Alarm auszugeben. Die Bewertungseinheit ist vorzugsweise als Mensch-Maschine-Interface (MMI) ausgestaltet. Ein ausgegebener Alarm kann visuell und/oder akustisch über das MMI übermittelt werden. Beispielsweise kann das MMI ein Display eine SMS oder E-Mail, ein 3-D Screen, eine Datenbrille oder eine Projektion ausgeben. Das MMI kann zudem eine automatische Anruffunktion oder Sprachansage ausgeben. Durch die Ausgestaltung der Bewertungseinheit als MMI kann bei einer negativen Prädiktion ein Alarm an den Betreuer übermittelt werden. Optional ist die Bewertungseinheit auch aktiv, wenn das Beatmungsgerät nicht therapiert oder ausgeschaltet ist. In der Regel ist die Logik der Bewertungseinheit veränderbar eingerichtet, wobei ein Eingriff in das Beatmungsgerät entfällt. Beispielsweise kann somit eine Konfiguration oder ein Update der Bewertungseinheit unabhängig vom Beatmungsgerät durchgeführt werden.

Die Informationseinheit ist in der Regel als Zugriffspunkt zur Zuführung zusätzlicher Informationen über den Anwender durch den Betreuer in die Telemonitoringeinheit des Systems eingerichtet. Beispielsweise können Informationen zum Krankheitstyp, zur Risikoklasse oder zur Anamnese für die Analyse über die Informationseinheit in die Telemonitoringeinheit eingebracht werden. Generell können alle über den direkten Kommunikationskanal ausgetauschten Informationen in das System eingebracht bzw. an die Telemonitoringeinheit durch die Informationseinheit übermittelt werden.

In Ausgestaltung ist die Informationseinheit als Dateneingabe eingerichtet. Durch die Informationseinheit sind Werte zum Zustand des Anwenders in die Telemonitoringeinheit einbringbar, wobei die Werte in der Speichereinheit der Telemonitoringeinheit gespeichert werden. Die Informationseinheit kann als digitale Schnittstelle, beispielsweise als Softwareapplikation, die auf einem Computer oder einem Tablett oder einem Smartphone ausgeführt werden kann, ausgebildet sein. Die Informationseinheit kann als MMI ausgebildet sein. Die Informationseinheit kann als Eingabedisplay oder als akustische Abfrage ausgestaltet sein. Über die Informationseinheit können beispielsweise Informationen über Antworten des Anwenders zu standardisierten Fragebögen, z.B. zu Atemnot, Quality of Life, Exazerbationsrisiko sein. Somit können mittels der Informationseinheit auch Informationen eines Betreuers in Form eines elektronischen Protokolls in das System eingebracht werden.

Auch kann ein Betreuer, beispielsweise ein Arzt, seine aktuellen Informationen über den Anwender und/oder Werte einer Visite über die Informationseinheit in die Telemonitoringeinheit eingeben, um die aktuellen Informationen den Analysen der Analyseeinheiten zugrunde zu legen. Die Informationseinheit bietet somit die Möglichkeit, Analysen, die in der Telemonitoringeinheit durchgeführt werden, personalisiert auf einen Anwender durchzuführen und jeweils die personenbezogenen aktuellen Aspekte eines Erkrankten bei der Analyse mitzuberücksichtigen.

In weiterer Ausgestaltung ist die Telemonitoringeinheit als Kombinationsschnittstelle ausgebildet, die eingerichtet ist, über die Informationsschnittstelle an die Telemonitoringeinheit übermittelte Werte und/oder Informationen, über die Eingabeeinheit übermittelte Werte und/oder Informationen sowie über das Beatmungsgerät an die Telemonitoringeinheit übermittelten Werte und/oder Informationen zu kombinieren und einer weiteren Analyse und/oder Bewertung zugrunde zu legen. Werte und/oder Informationen aus externen Datenquelle können dabei beispielsweise Werte zu Co2, z.B. ptCo2, SpO2, FiO2, Werte aus dem Insufflator/Exsufflator sowie Blutgase oder Blutwerte (Marker) sein. Die Ausgestaltung der Telemonitoringeinheit als Kommunikationsschnittstelle bietet den Vorteil, dass Messwerte und/oder Informationen verschiedener Quellen zusammenführbar sind, wobei die Quellen räumlich bzw. örtlich voneinander getrennt sein können. Werte und Informationen können dabei von Anwendern, Betreuern oder externen Datenquellen stammen. Die zusammengeführten Informationen können als Grundlage für Analysen genutzt werden. Analysen, die auf den zusammengeführten Werten und/oder Informationen der verschiedenen Quellen basiert, ermöglicht durch das Einfließen einer Vielzahl unterschiedlicher Werte eine genaue und personenbezogene Analyse.

In Ausgestaltung ist die Telemonitoringeinheit ein externer Server. Die Ausgestaltung der Telemonitoringeinheit als externer Server bietet den Vorteil, dass sowohl ein Anwender als auch einen Betreuer jeweils auf die Telemonitoringeinheit zugreifen und Informationen hinterlegen können, sodass personenbezogene Analysen und Bewertungen durchgeführt werden können, ohne dass der Anwender und oder der Betreuer direkt an dem Beatmungsgerät den Zugriff vornehmen muss.

In weiterer Ausgestaltung ist die Eingabeeinheit an dem Beatmungsgerät oder als externes Endgerät ausgebildet. Die Eingabeeinheit kann dabei in einem Display des Beatmungsgerätes integriert ausgebildet oder als Software in einer Applikation eingerichtet sein. Die als Softwareapplikation eingerichtete Eingabeeinheit kann beispielsweise auf Tabletts, Smartphones oder Computern ausgeführt werden. Besonders vorteilhaft ist die Ausführung der Eingabeeinheit als Applikation, da ein Patient ohne sich dem Beatmungsgerät zu nähern über ein Smartphone seinen Gesundheitszustand sowie die Konfiguration des Beatmungsgerätes prüfen kann und gegebenenfalls Einstellungen, die aufgrund der Analyse und Bewertung durch die Telemonitoringeinheit angezeigt werden vornehmen kann.

In Weiterbildung ist die Informationseinheit eingerichtet, externe Vergleichswerte und/oder Informationen aus externen Datenquellen zu beziehen. Beispielsweise kann die Informationseinheit eingerichtet sein, allgemeine Informationen zu einem Krankheitsbild aus einer Datenbank abzuprüfen und dem erfindungsgemäßen System zur Verfügung zu stellen. Externe Vergleichswerte und/oder Informationen können dabei Vergleichsmesswerte zu den durch das Beatmungsgerät erfassten Messwerten, Nebenwirkungen oder weiteren Hintergrundinformationen entsprechender Krankheitsbilder sein.

In einer weiteren Weiterbildung ist die mindestens eine Analyseeinheit der Telemonitoringeinheit eingerichtet, die in der Speichereinheit hinterlegten Werte des Anwenders und Werte über den Anwender zu kombinieren und zu analysieren, wobei die kombinierten Werte und Informationen zu zumindest einer Fragestellung in Beziehung gesetzt werden, wobei eine in einem definierten Zeitraum gegebene Erfüllung zumindest einer Fragestellung zumindest vorübergehend als Vorgang erfasst und über die mindestens eine Ausgabeeinheit ausgegeben wird. Dies bietet den Vorteil, dass sowohl aktuelle Informationen über den gefühlten und gemessenen Zustand des Anwenders als auch Hintergrundinformationen bzw. Fachinformationen eines Betreuers für die Analyse und Bewertung der durch das Beatmungsgerät ermittelten Messwerte sowie des ermittelten Gesundheitszustandes des Anwenders verwendet werden können.

In Ausgestaltung ist zumindest eine Ausgabeeinheit eingerichtet ist, bei Erfüllung der zumindest einen Fragestellung einen entsprechenden Alarm, vorzugsweise optisch und/oder akustisch, an die Eingabeeinheit auf dem Beatmungsgerät und/oder an ein Endgerät zu übermitteln und/oder eine Änderung der Arbeitsweise des Beatmungsgerätes zu bewirken, insbesondere der Parameter, mit der das Beatmungsgerät die Beatmung durchführt. Dabei ist wählbar, ob ein Alarm ausgegeben werden soll, wenn eine Fragestellung erfüllt oder nicht erfüllt ist. Beispielsweise kann ein entsprechender Alarm erfolgen, wenn die Fragestellung "Sitzt die Beatmungsmaske korrekt?" als nicht erfüllt angezeigt wird. In diesem Fall kann ein Alarm an das Beatmungsgerät oder an die Eingabeeinheit übermittelt werden, sodass der Anwender eine Korrektur des Sitzes der Maske vornehmen kann. Optional kann der Alarm durch Anweisungen zur Verbesserung, beispielsweise der Position der Beatmungsmaske, begleitet werden.

In einer Weiterbildung der Erfindung ist die Bewertungseinheit der Telemonitoringeinheit eingerichtet, aus mindestens zwei durch die Ausgabeeinheiten ausgegebenen Erfüllungen eine Gesamtkritikalität eines Gesundheitszustandes des Anwenders zu ermitteln und in Abhängigkeit der ermittelten Gesamtkritikalität einen Alarm auszugeben. Wahlweise kann die Bewertungseinheit eingerichtet sein, basierend auf ausgegebenen "Nicht-Erfüllungen" eine Gesamtkritikalität für die Qualität des Gesundheitszustandes des Anwenders zu ermitteln. Eine Gesamtkritikalität kann dabei durch fest hinterlegte Formeln, beispielsweise als Summen-Score oder Max-Score für Probleme hinterlegt sein. Optional können hinterlegte Formeln auswählbar und konfigurierbar eingerichtet sein. Zusätzlich können die Formeln durch Eingaben des Betreuers und/oder Anwenders zum Patiententyp (z.B. Erkrankung und Schweregrad) gewichtet werden.

In einer Ausgestaltung der Weiterbildung ist die Bewertungseinheit eingerichtet, basierend auf den Erfüllungen der Ausgabeeinheit eine Prädiktion in Bezug auf den Gesundheitszustand des Anwenders oder mögliche Komplikationen auszugeben. Alternativ kann die Prädiktion basieren auf ausgegebenen "Nicht-Erfüllungen" basieren. Die Logik der Prädiktion kann dabei in der Regel durch manuelle Änderung der Schwellenwerte oder durch automatisches Lernen anhand von Daten aus der Vergangenheit veränderbar sein. Optional kann die Bewertungseinheit eingerichtet sein, je nach ausgegebener Prädiktion einen für die jeweilige Prädiktion zugeteilten Betreuer zu alarmieren. In einer weiteren Ausführung ist ein aktueller Betreuer in der Bewertungseinheit hinterlegbar.

Die Erfindung betrifft ferner ein Verfahren der Anzeige oder Steuerung für ein System für die Beatmung nach einem der voranstehenden beschriebenen Merkmale, bei welchem in einem Beatmungsgerät durch mindestens einen Sensor einer Sensoreinheit Messwerte ermittelt werden, die eine Ermittlung von Druck und/oder Flow und/oder Volumen der zugeführten und/oder ausgeatmeten Gase erlauben, wobei diese Messwerte über eine Aufbereitungseinheit aufbereitet werden, zu weiteren Berechnungen einer Berechnungseinheit zugeführt werden und in einem weiteren Schritt zu weiteren Analysen einer Erkennungseinheit zugeführt werden, wobei das System die berechneten und analysierten Messwerte an eine Telemonitoringeinheit übersendet, und bei dem über eine Eingabeeinheit des Beatmungsgerätes durch einen Anwender Informationen zum Gesundheitszustand seiner Person hinterlegbar sind, wobei diese Informationen an die Telemonitoringeinheit übermittelt werden.

Erfindungsgemäß setzt die Telemonitoringeinheit die von der Kommunikationseinheit des Beatmungsgerätes übermittelten Messwerte, Signale und Kenngrößen zu über eine Informationseinheit der Telemonitoringeinheit durch einen Betreuer hinterlegten Informationen zum Gesundheitszustand des Anwenders und/oder externen Vergleichswerten in Beziehung und prüft zumindest hinsichtlich einer Fragestellung. Die Telemonitoringeinheit übermittelt Werte aus dem Beatmungsgerät, aus der Eingabeeinheit, aus einer zusätzlichen Messeinheit, aus einer zusätzlich Telemonitoringeinheit an eine Kommunikationseinheit der Telemonitoringeinheit, wobei die übermittelten Werte in einer Speichereinheit zwischengespeichert und an zumindest eine Analyseeinheit, die in der Telemonitoringeinheit ausgebildet ist, weitergeleitet werden. Die zumindest eine Analyseeinheit prüft die übermittelten Werte hinsichtlich zumindest einer Fragegestellung.

In Ausgestaltung gibt zumindest eine Ausgabeeinheit bei Erfüllung zumindest einer durch zumindest eine Analyseeinheit dargestellten Fragestellung einen entsprechenden Alarm und/oder eine entsprechende Anzeige aus. Alternativ kann durch die zumindest eine Ausgabeeinheit bei Nicht-Erfüllung der Fragestellung ein entsprechender Alarm ausgegeben werden.

In weiterer Ausgestaltung erhält der Betreuer bei Erfüllung zumindest einer Fragestellung eine Information darüber, welche Fragestellung erfüllt wurde und/oder welcher Parameter eine Abweichung vom Zielwert aufweist bzw. welche der Einstellungen Druck, Flow, Volumen, Zeit, Frequenz angepasst werden müssten, um wieder in einen vorgegebenen Zielwertbereich zu kommen. Die Information wird vorzugsweisen über die Bewertunsgeinheit an den Betreuer übermittelt. Alternativ kann der Betreuer bei Nicht-Erfüllung zumindest einer Fragestellung eine Information darüber, welche Fragestellung erfüllt wurde und/oder welcher Parameter eine Abweichung vom Zielwert aufweist bzw. welche der Einstellungen Druck, Flow, Volumen, Zeit, Frequenz angepasst werden müssten.

In einer Weiterbildung des Verfahrens werden die Informationen über die Zielwertabweichung und/oder eine erfüllte Fragestellung zusammen mit Empfehlungen hinsichtlich der notwendigen Anpassung eines oder mehrerer Parameter im Beatmungsgerät abgespeichert werden und dort durch den Anwender abgerufen werden und auf Auswahl hin auch automatisch ausgeführt werden oder über die Bewertungseinheit der Telemonitoringeinheit dem Betreuer übermittelt werden. Optional können notwendige Einstellungen an dem Beatmungsgerät durch den Betreuer basierend auf den an den Betreuer ausgegebenen Informationen über eine Fernübertragung durchgeführt werden.

In Ausgestaltung ermittelt eine Bewertungseinheit der Telemonitoringeinheit aus den erfüllten Fragestellungen eine Prädiktion in Bezug auf den Gesundheitszustand des Anwenders und/oder Komplikationen. Beispielsweise kann eine Prädiktion zum Gesundheitszustand ausgegeben werden, wenn zumindest zwei von vier Fragestellungen als erfüllt ausgegeben werden. Optional kann eine Prädiktion auch für weitere Kategorien erfolgen. Eine Prädiktion kann beispielsweise eine Information über eine zunehmend schnellere und flachere Atmung sein. Alternativ gibt die Bewertungseinheit basierend auf zumindest einer nicht erfüllten Fragestellung eine Prädiktion über den Verlauf der Messwerte, den Gesundheitszustand oder mögliche auftretende Komplikationen aus. In der Regel ist die Ausgabe der Prädiktion basierend auf einer Erfüllung oder Nicht-Erfüllung wählbar.

Optional werden die Informationen über die Zielwertabweichung und/oder erfüllte Fragestellung zusammen mit Empfehlungen hinsichtlich der notwenigen Anpassung eines oder mehrerer Parameter auch vom Beatmungsgerät über Fernübertragung (Internet, Funk, Kabel o.ä.) zu einem Arzt übermittelt, woraufhin dieser optional ebenfalls über eine Fernübertragung die notwendigen Einstellungen der Parameter anpasst.

Die vorliegende Erfindung betrifft ferner ein Beatmungsgerät umfassend ein System für die Beatmung nach einem der vorgenannten Merkmale.

Im Folgenden werden anhand von stark vereinfachten schematischen Darstellungen bevorzugte Ausführungsbeispiele der Erfindung näher erläutert. Es zeigt:
- Fig. 1: eine Ausführungsform einer Ausgestaltung des erfindungsgemäßen Systems für die Beatmung,
- Fig. 2: eine Darstellung einer Ausführungsform einer Wiedergabe einer Anzeige.

In den Figuren weisen dieselben konstruktiven Elemente jeweils dieselben Bezugsziffern auf.

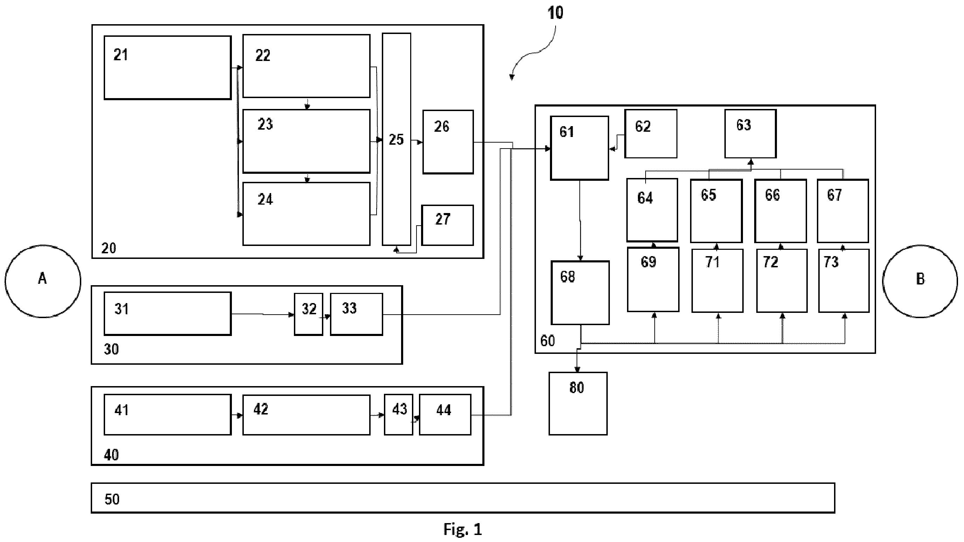

In der Figur 1 ist eine Ausführungsform des erfindungsgemäßen Systems für die Beatmung 10 dargestellt.

Das erfindungsgemäße System 10 umfasst in diesem Ausführungsbeispiel ein Beatmungsgerät 20 sowie eine Eingabeeinheit 30, eine zusätzliche Messeinheit 40, einen direkten Kommunikationskanal 50, eine Telemonitoringeinheit 60 sowie eine zusätzliche Telemonitoringeinheit 80.

Das erfindungsgemäße System 10 ist eingerichtet, die Kommunikation zwischen einem Anwender A und einem Betreuer B zu erleichtern und zu verbessern sowie die Qualität der Beatmungstherapie zu verbessert. Ein Betreuer kann beispielsweise ein Arzt, ein Therapeut, ein Home Care Provider, ein Pfleger oder ein Angehöriger sein.

Das Beatmungsgerät 20 umfasst in der vorliegenden Ausführungsform eine Sensoreinheit 21, eine Aufbereitungseinheit 22, eine Berechnungseinheit 23, eine Erkennungseinheit 24, eine Zwischenspeichereinheit 25, eine Kommunikationseinheit 26, sowie eine Überwachungseinheit 27.

Die Sensoreinheit 21 ist eingerichtet Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen. Optional kann die Sensoreinheit 21 zusätzliche Messungen von Bestandteilen oder Temperatur des Atemgases oder des Blutes vornehmen. Die Sensoreinheit 21 übermittelt die erfassten Messwerte an die Aufbereitungseinheit 22.

Die Aufbereitungseinheit 22 kann die erfassten Messwerte aufbereiten. Beispielsweise kann die Aufbereitungseinheit eine Glättung, eine Artefaktbereinigung oder ein Downsampling der Messwerte durchführen. Die Aufbereitungseinheit übermittelt die aufbereiteten Messwerte an die Berechnungseinheit 23.

Die Berechnungseinheit 23 berechnet aus den durch die Sensoreinheit 21 erfassten und durch die Aufbereitungseinheit 22 aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten. Die Berechnungseinheit 23 übermittelt die berechneten Messwerte, Signale und Kenngrößen an die Erkennungseinheit 24.

Die Erkennungseinheit 24 ist eingerichtet, Ereignisse/Zustände wie beispielsweise Alarme, Atemaussetzer, Artefakte, Hustenstöße, Sauerstoffentsättigungen, asynchrone zwischen Gerät und Anwender, Einatmen, Ausatmen oder mandatorische Atemzüge zu erkennen.

Die Zwischenspeichereinheit 25 eingerichtet die durch die Berechnungseinheit 23 berechneten Signale und Kenngrößen und/oder die durch die Erkennungseinheit 24 erkannten Ereignisse zwischenzuspeichern. Vorteilhafterweise speichert die Zwischenspeichereinheit 25 die gespeicherten Informationen zumindest bis zu einem nächsten Kommunikationsvorgang zwischen der Kommunikationseinheit 26 des Beatmungsgerätes 20 und der Telemonitoringeinheit 60. Die Kommunikationseinheit 26 ist beispielsweise eines der folgenden genannten Kommunikationssysteme: GSM, Lora, LPWAN, NB-IOT, LTE-M, LTE, UMTS, LAMN WIFI, WLAN, Bluetooth.

Zudem kann die Überwachungseinheit 27 technische Probleme des Beatmungsgerätes 20 erfassen. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Überwachungseinheit 27 kann bei einem erkannten technischen Problem einen Alarm auf dem Beatmungsgerät anzeigen oder über die Telemonitoringeinheit 60 an den Betreuer übermitteln.

Die Eingabeeinheit 30 des Systems für die Beatmung 10 ist ein Mensch- Maschine-Interface 31, dass eine Eingabe von Messwerten und/oder Informationen eines Anwenders an das Beatmungsgerät ermöglicht. Beispielsweise kann so ein Anwender Information anderer Messgeräte wie beispielsweise eine mittels eines Thermometers gemessenen Temperatur oder eines mittels eines Pulsmessgerätes erfassten Pulses in das System 10 einbringen. Die so eingebrachten Messwerte und/oder Informationen können direkt durch die Eingabeeinheit 30 oder durch das Beatmungsgerät 20 an die Telemonitoringeinheit 60 übermittelt werden und dort für Analysen genutzt werden.

Die Eingabeeinheit 30 umfasst zudem eine Zwischenspeichereinheit 32 sowieso eine Kommunikationseinheit 33. Die Zwischenspeichereinheit 32 speichert die durch den Anwender eingegebenen Informationen in der Eingabeeinheit 30. Die Kommunikationseinheit 33 ist analog zu der Kommunikationseinheit 26 in dem Beatmungsgerät ausgebildet. Die Kommunikationseinheit 33 übermittelt die eingegebenen Werte an die Telemonitoringeinheit 60. Alternativ zu einer direkten Kommunikation zur Telemonitoringeinheit 60 kann die Übermittlung der Werte zunächst an das Beatmungsgerät 20 erfolgen. Beispielsweise kann diese Kommunikation zwischen der Eingabeeinheit 30 und dem Beatmungsgerät 20 über ein Kabel oder WiFi oder Bluetooth erfolgen. Die Kommunikationseinheit 33 kann die zwischengespeicherten Werte der Eingabeeinheit 30 gebündelt oder kontinuierlich an das Telemonitoringsystem 60 oder das Beatmungsgerät 20 weiterleiten.

Die vorliegende Ausführungsform des erfindungsgemäßen Systems 10 für die Beatmung beinhaltet zudem eine zusätzliche Messeinheit 40, die eine Sensoreinheit 41, eine Verarbeitungseinheit 42, einen Zwischenspeicher 43 und für eine Kommunikationseinheit 44 umfasst. Die zusätzliche Messeinheit 40 kann optional in dem erfindungsgemäßen System 10 für die Beatmung ausgebildet sein. In einer alternativen Ausführungsform kann das erfindungsgemäße System 10 auf eine zusätzliche Messeinheit 40 verzichten. Die zusätzliche Messeinheit 40 kann beispielsweise ein Messgerät zur Erfassung von Sauerstoff, einer Sauerstoffsättigung im Blut, Atemgasen, CO2 im Blut, eines Blutdrucks, einer Herzfrequenz, Husten, Sekret, Körpertemperatur, Gewicht oder einer Aktivität sein. Die zusätzliche Messeinheit 40 umfasst dabei vorzugsweise eine Sensoreinheit 41, wobei die Sensoreinheit 41 zumindest ein Sensor zum Beispiel für die Erfassung von O2, SpO2, Co2, Temperatur, Gesichts, Herzfrequenz, Husten, Atemgase, Blutdruck oder Aktivität ist. Die zusätzliche Messeinheit 40 kann auch ein Aktivitätsmessgerät, ein Schlafqualitätsmessgerät oder ein Lungenfunktionsmessgerät sein. Die Verarbeitungseinheit 42 verarbeitet die ermittelten Messwerte zum Beispiel durch Downsampling, Glättung oder Artefakterkennung, Berechnung statistischer Kennzahlen oder durch Erkennung von Zuständen und Ereignissen. Der Zwischenspeicher 42 speichert die verarbeiteten Messwerte. Die mittels der zusätzlichen Messeinheit 40 ermittelten Messwerte können über die Kommunikationseinheit 44 an die Telemonitoringeinheit 60 übermittelt werden und weiteren Analysen zugrunde gelegt werden. Die Kommunikationseinheit 44 ist analog zu der Kommunikationseinheit 26 des Beatmungsgerätes 20 ausgebildet. Die Kommunikationseinrichtung 44 kann die verarbeiteten Messwerte beispielsweise via GSM, Lora, LPWAN, NB-IOT, LTE-M, UMTS, LAN, WIFI, WLAN oder Bluetooth an die Telemonitoringeinheit 60 übermitteln. Alternativ zu einer direkten Verbindung zu der Telemonitoringeinheit 60 kann auch eine Kommunikation mit dem Beatmungsgerät 20 beispielsweise über ein Kabel, WiFi oder Bluetooth erfolgen. Dabei werden die Informationen gebündelt an die Telemonitoringeinheit 60 weiter übermittelt.

Das System für die Beatmung 10 kann auch einen direkten Kommunikationskanal 50 umfassen. Der direkte Kommunikationskanal 50 kann beispielsweise per Sprache oder Video erfolgen und für eine Abfrage einer Symptomatik genutzt werden. Über den direkten Kommunikationskanal 50 können Beschwerden, eine Quality of Life, Risk Scores, Nebenwirkungen, Fragen zur Therapie, Klärung von handhabungs-Schwierigkeiten sowie Anweisungen im Falle von Problemsituationen oder manuelle Notfall-Alarme zwischen Anwender und Betreuer übermittelt werden. Die Informationen aus dem direkten Kommunikationskanal 50 können in die Telemonitoringeinheit 60 eingegeben werden und für weitere Analysen genutzt werden.

Die Telemonitoringeinheit 60 des Systems 10 umfasst eine Kommunikationseinheit 61, eine Informationseinheit 62, eine Bewertungseinheit 63, Ausgabeeinheiten 64, 65, 66, 67, eine Speichereinheit 68, sowie Analyseeinheiten 69, 71, 72, 73.

Die Kommunikationseinheit 61 fungiert in der vorliegenden Ausführungsform als Puffer zur Kommunikation mit einer Vielzahl von Beatmungsgeräten. Die Kommunikationseinheit 61 empfängt die durch das Beatmungsgerät 20, die Eingabeeinheit 30 und das zusätzliche Messgeräte 40 übermittelten Messwerte.

Die Speichereinheit 68 ist eingerichtet, die über die Kommunikationseinheit 61 an die Telemonitoringeinheit 60 übermittelten Wert zumindest zwischenzuspeichern. Ferner kann die Speichereinheit 68 eine Zuordnung eines Anwenders zu einem Gerät, beispielsweise über den Namen des Anwenders, eine ID oder eine Seriennummer durchführen.

Die Informationseinheit 62 ist als Dateieingabe eingerichtet und ermöglicht eine Eingabe zusätzlicher Werte und Informationen durch den Betreuer. Beispielsweise können Informationen zu Krankheitstypen, Informationen zu einer Risikoklasse, Informationen zur Anamnese, sowie alle über den direkten Kommunikationskanal 50 ausgetauschten Informationen in die Telemonitoringeinheit 60 eingebracht werden ebenso wie Interventionsprotokolle übertragen werden. Entsprechend der Informationen zu Krankheitstypen, Informationen zu einer Risikoklasse, Informationen zur Anamnese können jeweils unterschiedliche spezifische Analyse-Regeln gelten und (von der Analyseeinheit) bereitgestellt und angewendet werden.

Über die Informationseinheit 62 können zusätzliche Informationen und Messwerte über den Anwender durch den Betreuer in das System eingebracht werden, um diese bei der Analyse zu berücksichtigen. Beispielsweise können Informationen über einen Krankheitstyp, zu einer Risikoklasse oder zur Anamnese über die Informationseinheit eingebracht werden. Generell können jegliche Informationen, die beispielsweise auch über einen direkten Kommunikationskanal 50 ausgetauscht werden können oder Interventionsprotokolle ebenfalls über die Inflationseinheit der Telemonitoringeinheit 60 zur weiteren Analyse bereitgestellt werden.

Die Analyseeinheiten 69, 71, 72, 73 sind eingerichtet, Analysen in Bezug auf zumindest eine Fragestellung auszuführen. Mögliche Fragestellungen können dabei sein, ob das Beatmungsgerät ausreichend genutzt wird, ob eine ausreichende Dichtigkeit von Mund und Patienteninterface/Beatmungsmaske vorliegt, ob das Beatmung Gerät korrekt funktioniert und konfiguriert ist bzw. ob die Einstellungen des Beatmungsgerätes korrekt sind, ob der Krankheitszustand stabil ohne Anzeichen einer Exspiration/Dekompensation ist. Für jede Fragestellung kann ein Indikator, beispielsweise "gut" oder "schlecht", 0 und 100% Problemstärke, 0 und 100% Therapiegüte oder 3-5 Kategorien wie beispielsweise "sehr gut", "gut", "mittel", "schlecht" und "sehr schlecht" verwendet werden. Optional können die Indikatoren über alle Fragestellungen in einer weiteren Stufe addiert/kombiniert werden. Dies bietet den Vorteil, dass Anwender mit mehreren "Problemen" getrennt zu Anwendern eines "Problems" darstellbar sind. Optional kann eine Gewichtung der Probleme vorgenommen werden.

Die Ausgabeeinheiten 64, 65, 66, 67 sind eingerichtet, die Ausgabe von Analyseergebnissen der mindestens zwei Analyseeinheiten beispielsweise in Form einer Kennzahl oder einer Grafik und farbliche Differenzierung mindestens gute und schlechte Ergebnisse zu ermöglichen.

Die Bewertungseinheit 63 ermittelt basierend auf der Anzahl der durch die Ausgabeeinheit ausgegebenen Ergebnisse eine Gesamtbewertung über den Gesundheitszustand des Anwenders. Optional kann eine aktive Benachrichtigung des Betreuers per Chat, E-Mail, SMS, Anruf oder über einen weiteren Kommunikationsweg erfolgen.

Ferner ermittelt die Bewertungseinheit 63, basierend auf zumindest einer als erfüllt oder nicht erfüllt bewerteten Fragestellung der Ausgabeeinheit 64, 65, 66, 67 eine Prädiktion über den Verlauf der Messwerte, den Gesundheitszustand des Anwenders oder mögliche auftretende Komplikationen. Beispielsweise wird basierend auf zumindest zwei als erfüllt bewerteten Fragestellungen eine Prädiktion über den Gesundheitszustand oder auftretende Komplikationen ausgegeben. Alternativ ist die Bewertungseinheit 63 eingerichtet, basierend auf zumindest einer nicht erfüllten Fragestellung eine Prädiktion über den Verlauf der Messwerte, den Gesundheitszustand oder mögliche auftretende Komplikationen auszugeben. In der Regel ist die Ausgabe der Prädiktion basierend auf einer Erfüllung oder Nicht-Erfüllung wählbar.

Die Bewertungseinheit 63 kann basierend auf der Prädiktion, beispielsweise einer Komplikation, einen Alarm auszugeben. Die Bewertungseinheit 63 ist vorzugsweise als Mensch-Maschine-Interface (MMI) ausgestaltet. Ein ausgegebener Alarm kann visuell und/oder akustisch über das MMI übermittelt werden. Das MMI kann zudem eine automatische Anruffunktion oder Sprachansage ausgeben. Durch die Ausgestaltung der Bewertungseinheit 63 als MMI kann bei einer negativen Prädiktion ein Alarm an den Betreuer übermittelt werden.

Das System 10 umfasst in dem vorliegenden Ausführungsbeispiel zudem eine weitere Telemonitoringeinheit 80. Die weitere Telemonitoringeinheit 80 kann beispielsweise ein Server mit Software zur Verknüpfung mit weiteren Informationen umfassen. Die Kommunikation kann beispielsweise über API, SFTP, https oder Dateien erfolgen. Auch andere Kommunikationswege sind kompatibel/verwendbar. Die Werte der zusätzlichen Telemonitoringeinheit 80 werden an die Telemonitoringeinheit 60 übermittelt und den weiteren Analysen zugrunde gelegt.

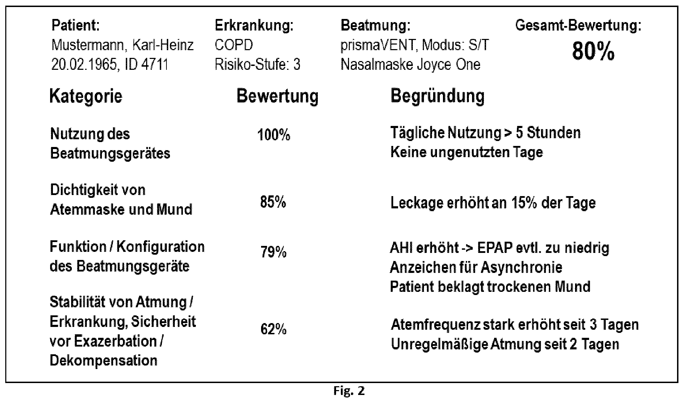

In der Figur 2 ist eine Ausführungsform einer beispielhaften Anzeige dargestellt. Eine derartige Anzeige kann beispielsweise auf dem Beatmungsgerät oder einem Endgerät angezeigt werden. Dabei ist beispielsweise der Patient mit Namen, Geburtsjahr und oder ID identifiziert. Zudem können Angaben angezeigt werden bezüglich der Erkrankung, beispielsweise eine COPD Erkrankung sowie eine Risikostufe. Zudem kann angezeigt werden, in welcher Form in welchem Modus der Patient derzeit beatmet wird, mit welchem Gerät und mit welchem Zubehörmaterial. Des Weiteren können verschiedene Kategorien, Bewertungen zu diesen Kategorien und eine zusätzliche Begründung angezeigt werden. Beispiel für eine Kategorie können sein die Nutzung des Beatmungsgerätes, die Dichtigkeit Atemmaske und Mund, eine Funktion/Konfiguration des Beatmungsgerätes oder eine Stabilität der Atmung/Erkrankung bzw. eine Sicherheit vor Exazerbation/Dekompensation. Die vorgenannten Kategorien können über die Bewertung dargestellt werden. Dabei kann beispielsweise eine Einteilung von 0-100 % dargestellt werden. In der Begründung können zusätzliche Hilfsinformationen dem Anwender und oder dem Betreuer angezeigt werden. Ferner ist in der vorliegenden Ausführungsform einer beispielhaften Anzeige eine Gesamtbewertung angegeben. Vorliegend wird die Gesamtbewertung Beispiel achten 80% angegeben. Die Gesamtbewertung liefert eine Aussage über den Gesamtgesundheitszustand des Anwenders. Die grafische Darstellung der Anzeige ist variabel und kann durch einen Anwender und oder Betreuer angepasst werden. Optional können weitere Kategorien entsprechend des Anwenders der Anzeige hinzugefügt werden. Optional kann die Bewertung grafisch dargestellt werden. In der Regel umfasst die Anzeige zudem bei Ausgabe einer Erfüllung bzw. Nicht-Erfüllung einer Fragestellung durch die -in Fig. 1- gezeigte Ausgabeeinheit eine Prädiktion in Bezug zu dem Gesundheitszustand des Anwenders oder ggf. zukünftig auftretenden Komplikationen an.

### Bezugszeichenliste

A Anwender
B Betreuer
10 System für die Beatmung
20 Beatmungsgerät
21 Sensoreinheit
22 Aufbereitungseinheit
23 Berechnungseinheit
24 Erkennungseinheit
25 Zwischenspeichereinheit
26 Kommunikationseinheit
27 Überwachungseinheit
30 Eingabeeinheit
31 Mensch-Maschine-Interface
32 Zwischenspeichereinheit
33 Kommunikationseinheit
40 zusätzliche Messeinheit
41 Sensoreinheit
42 Verarbeitungseinheit
43 Zwischenspeicher
44 Kommunikationseinheit
50 direkter Kommunikationskanal
60 Telemonitoringeinheit
61 Kommunikationseinheit
62 Informationseinheit
63 Bewertungseinheit
64, 65, 66, 67 Ausgabeeinheiten
68 Speichereinheit
69, 71, 72, 73 Analyseeinheiten
80 zusätzliche Telemonitoringeinheit

## Patentansprüche

1. System (10) für die Beatmung umfassend zumindest ein Beatmungsgerät (20), zumindest eine Eingabeeinheit (30) und zumindest eine Telemonitoringeinheit (60), wobei das mindestens eine Beatmungsgerät (20) mindestens eine Sensoreinheit (21), eine Aufbereitungseinheit (22), eine Berechnungseinheit (23), eine Erkennungseinheit (24), eine Speichereinheit (25) und eine Kommunikationseinheit (27) umfasst, wobei die Sensoreinheit (21) Sensoren umfasst, deren Messwerte eine Ermittlung von Druck und/oder Flow und/oder Volumen der zugeführten und/oder ausgeatmeten Gase erlauben, die Aufbereitungseinheit (22) die erfassten Messwerte aufbereitet, die Berechnungseinheit (23) basierend auf den Messwerten Signale und/oder Kenngrößen bestimmt und die Speichereinheit (25) diese Werte speichert und die Erkennungseinheit (24) die Werte zu weiteren Analysen nutzt, wobei die Eingabeeinheit (30) eingerichtet ist, durch einen Anwender (A) des Systems (10) über die Eingabeeinheit (30) eingegebene Werte und Informationen des Anwenders (A) zu seinem Gesundheitszustand dem System (10) bereitzustellen, und wobei die Kommunikationseinheit (26) die Werte an die Telemonitoringeinheit (60) übermittelt, **wobei** die Telemonitoringeinheit (60) eingerichtet ist, die übermittelten Werte und/oder Informationen des Beatmungsgerätes (20) und der Eingabeeinheit (30) des Beatmungsgerätes zu empfangen, zu speichern, zu analysieren und/oder zu bewerten
**dadurch gekennzeichnet, dass** die mindestens eine Analyseeinheit (69, 71, 72, 73) der Telemonitoringeinheit (60) eingerichtet ist, die in der Speichereinheit (68) hinterlegten Werte und Informationen des Anwenders und Informationen über den Anwender zu kombinieren und zu analysieren, wobei die kombinierten Werte und Informationen zu zumindest einer Fragestellung in Beziehung gesetzt werden, wobei eine in einem definierten Zeitraum gegebene Erfüllung zumindest einer Fragestellung zumindest vorübergehend als Vorgang erfasst und über mindestens eine Ausgabeeinheit (64, 65, 66, 67) ausgegeben wird, wobei zumindest eine Ausgabeeinheit eingerichtet ist, bei Erfüllung der zumindest einen Fragestellung einen entsprechenden Alarm, an die Eingabeeinheit (30) auf dem Beatmungsgerät (20) zu übermitteln und/ eine Änderung der Arbeitsweise des Beatmungsgerätes (20) zu bewirken, insbesondere der Parameter, mit der das Beatmungsgerät (20) die Beatmung durchführt, wobei Informationen über eine Zielwertabweichung für **die Parameter** Druck, Fluss, Volumen, Zeit, Frequenz oder eine erfüllte Fragestellung mit Empfehlungen hinsichtlich der notwendigen Anpassung mehrerer der Parameter Druck, Fluss, Volumen, Zeit, Frequenz im Beatmungsgerät
abgespeichert werden und dort durch den Anwender abgerufen werden und auf Auswahl hin auch automatisch ausgeführt werden und notwendige Einstellungen an dem Beatmungsgerät durch den Betreuer basierend auf den an den Betreuer ausgegebenen Informationen über eine Fernübertragung durchgeführt werden können.

2. System (10) nach einem der voranstehenden Ansprüche, **dadurch gekennzeichnet, dass** die Telemonitoringeinheit (60) eine Kommunikationseinheit (61), eine Speichereinheit (68), zumindest eine Analyseeinheit (69, 71, 72, 73), zumindest eine Ausgabeeinheit (64, 65, 66, 67), eine Bewertungseinheit (63) und zumindest eine Informationseinheit (62), umfasst.

3. System (10) nach einem der voranstehenden Ansprüche, **dadurch gekennzeichnet, dass** die Informationseinheit (62) der Telemonitoringeinheit (60) als Dateneingabe eingerichtet ist.

4. System (10) nach Anspruch 1, **dadurch gekennzeichnet, dass** die Telemonitoringeinheit (60) als Kombinationsschnittstelle ausgebildet ist, die eingerichtet ist, über die Informationsschnittstelle an die Telemonitoringeinheit (60) übermittelte Werte und/oder Informationen, über die Eingabeeinheit (30) übermittelte Werte und/oder Informationen sowie über das Beatmungsgerät (20) an die Telemonitoringeinheit (60) übermittelten Werte und/oder Informationen zu kombinieren und zumindest einer weiteren Analyse und/oder Bewertung zugrunde zu legen.

5. System (10) nach Anspruch 1 oder 2, **dadurch gekennzeichnet, dass** die Telemonitoringeinheit (60) ein externer Server ist.

6. System (10) nach einem der voranstehenden Ansprüche, **dadurch gekennzeichnet, dass** die Eingabeeinheit (30) des Beatmungsgerätes an dem Beatmungsgerät (20) oder als externes Endgerät ausgebildet ist.

7. System (10) nach einem der voranstehenden Ansprüche, **dadurch gekennzeichnet, dass** die Informationseinheit (62) eingerichtet ist, externe Vergleichswerte und/oder Informationen aus externen Datenquellen zu beziehen.

8. System (10) nach einem der voranstehenden Ansprüche, **dadurch gekennzeichnet, dass** die Bewertungseinheit (63) der Telemonitoringeinheit (60) eingerichtet ist, aus mindestens zwei durch die Ausgabeeinheiten (64, 65, 66, 67) ausgegebenen Erfüllungen eine Gesamtkritikalität eines Gesundheitszustandes des Anwenders zu ermitteln und in Abhängigkeit der ermittelten Gesamtkritikalität einen Alarm auszugeben.

9. System (10) nach einem der voranstehenden Ansprüche, **dadurch gekennzeichnet, dass** die Bewertungseinheit (63) eingerichtet ist, basierend auf den Erfüllungen der Ausgabeeinheit (64, 65, 66, 67) eine Prädiktion in Bezug auf den Gesundheitszustand des Anwenders oder mögliche Komplikationen auszugeben.

10. Verfahren der Anzeige oder Steuerung für ein System für die Beatmung nach einem der Ansprüche 1 bis 9, bei welchem in einem Beatmungsgerät (20) durch mindestens einen Sensor einer Sensoreinheit (21) Messwerte ermittelt werden, die eine Ermittlung von Druck und/oder Flow und/oder Volumen der zugeführten und/oder ausgeatmeten Gase erlauben, wobei diese Messwerte über eine Aufbereitungseinheit (22) aufbereitet werden, zu weiteren Berechnungen einer Berechnungseinheit (23) zugeführt werden und in einem weiteren Schritt zu weiteren Analysen einer Erkennungseinheit (24) zugeführt werden, wobei das System (10) die berechneten und analysierten Messwerte an eine Telemonitoringeinheit (60) übersendet, und bei dem über eine Eingabeeinheit (30) des Beatmungsgerätes (20) durch einen Anwender Informationen zum Gesundheitszustand seiner Person hinterlegbar sind, wobei diese Informationen an die Telemonitoringeinheit (60) übermittelt werden, **dadurch gekennzeichnet,** die Telemonitoringeinheit (60) die von der Kommunikationseinheit (26) des Beatmungsgerätes (20) übermittelten Messwerte, Signale und Kenngrößen zu über eine Informationseinheit (62) der Telemonitoringeinheit (60) durch einen Betreuer hinterlegten Informationen zum Gesundheitszustand des Anwenders und/oder externen Vergleichswerten in Beziehung setzt und zumindest hinsichtlich einer Fragestellung prüft.

11. Verfahren nach Anspruch 10, **dadurch gekennzeichnet, dass** zumindest eine Ausgabeeinheit (64, 65, 66, 67) bei Erfüllung zumindest einer durch zumindest eine Analyseeinheit (69, 71 ,72 ,73) dargestellten Fragestellung einen entsprechenden Alarm und/oder eine entsprechende Anzeige ausgibt.

12. Verfahren nach Anspruch 10 oder 11, **dadurch gekennzeichnet, dass** der Betreuer bei Erfüllung zumindest einer Fragestellung eine Information darüber erhält, welche Fragestellung erfüllt wurde und/oder welcher Parameter eine Abweichung vom Zielwert aufweist bzw. welche der Einstellungen Druck, Flow, Volumen, Zeit, Frequenz angepasst werden müssten, um wieder in einen vorgegebenen Zielwertbereich zu kommen.

13. Verfahren nach Anspruch 12, **dadurch gekennzeichnet, dass** die Informationen über die Zielwertabweichung und/oder eine erfüllte Fragestellung zusammen mit Empfehlungen hinsichtlich der notwendigen Anpassung eines oder mehrerer Parameter im Beatmungsgerät (20) abgespeichert werden und dort durch den Anwender abgerufen werden und auf Auswahl hin auch automatisch ausgeführt werden oder über die Bewertungseinheit (63) der Telemonitoringeinheit (60) dem Betreuer übermittelt werden. (optional ebenfalls über eine Fernübertragung die notwendigen Einstellungen der Parameter anpasst.)

14. Verfahren nach einem der Ansprüche 10 bis 13, **dadurch gekennzeichnet, dass** eine Bewertungseinheit (63) der Telemonitoringeinheit (60) aus den erfüllten Fragestellungen eine Prädiktion in Bezug auf den Gesundheitszustand des Anwenders und/oder Komplikationen ermittelt.

15. Beatmungsgerät (20) umfassend ein System (10) für die Beatmung nach einem der Ansprüche 1 bis 9.
